# EUROPEAN PATENT APPLICATION

(11) **EP 2 813 264 A1**
(43) Date of publication of application: **17.12.2014**
(21) Application number: 13742913.0
(22) Date of filing: 23.01.2013
(51) Int. Cl.: A61Q 17/04, A61K 8/19

(54) **UV PROTECTION AGENT CONTAINING BISMUTH OXYCHLORIDE**

(30) Priority: 31.01.2012 MX 2012001424
(71) Applicant: Farmaquimia, S.A. de C.V, 54730 Cuautitlan Izcalli (MX)
(72) Inventor: BEREA LAGARDA, Gustavo Adolfo, C.P. 54730 Cuautitlan Izcalli Estado de México (MX); BEREA MONTES, Edgardo, C.P. 54730 Cuautitlan Izcalli Estado de México (MX); PARRA CERVANTES, Patricia, C.P. 15000 México D.F. (MX); SOTO VAZQUEZ, Ramón, C.P. 15000 México D.F. (MX); ROMERO CASTELLO, Samuel, C.P. 15000 México D.F. (MX)
(74) Representative: Stiebe, Lars Magnus
(86) International application number: PCT/MX2013/000009
(87) International publication number: WO 2013/115632

(57) **Abstract**

This invention refers to the use of a Bismuth inorganic salt, specifically, Bismuth Oxychloride as sun protection agent, and to the synthesis methods to obtain optimized Bismuth Oxychloride with specific photoprotecting features ensuring optimum stability, effectiveness and safety for the protection against skin and/or hair, and/or facial hair, and/or body hair damage caused by UV solar radiation. Furthermore, different compositions with effective and safe concentrations of Bismuth Oxychloride are described. These compositions exhibit protection values above those commonly obtained from organic and inorganic agents known in the state of the art, all along the ultraviolet spectrum (290 to 400 nm). This physically and chemically characterized compound, with critical wavelengths above 380 nm, is a "broad spectrum" sunscreen.

## Description

### FIELD OF THE INVENTION

This invention refers to the use of Bismuth Oxychloride, an inorganic compound, as a wide spectrum protection agent for the skin and/or hair and/or facial hair and/or body hair, against sun radiation along all the ultraviolet range (200 to 400 nm), achieving significantly better protection results than those of the agents conventionally used. This invention also refers to Bismuth Oxychloride compositions in specific concentrations and with specific physical and chemical characteristics, and to the preparation methods for such compositions that provide greater degree of efficacy and efficiency in sun protection, and specifically protection against ultraviolet rays.

### BACKGROUND OF THE INVENTION

Protecting the skin from sun radiation has been a common practice dating back to ancient times. The Egyptians had already identified and used natural substances such as olive oil to prevent sunburn induced by overexposure. Similar records are also found in the Greek and Roman civilizations. By the end of the 19^{th} Century, the first scientific report of a "photoprotecting" substance appears, but it was not until 1928 that a commercial composition based on benzyl cinnamate and salicylate appears specifically designed to that aim *(Lim and Draelos, 2009; Henschke, 1940*)*.*

The dramatic increase in skin cancer cases seen from the beginning of the 20^{th} Century to this date, apparently resulting from recreational habits of light-skinned people who move more frequently to sub-tropical regions, promoted the study and commercialization of sunscreens, expanding the knowledge about the effect of sun radiation on the skin. Today a reasonably good understanding of the mechanisms involved exists, and we know that ultraviolet rays are, by far, the most abundant carcinogen in the environment, and to which most people are exposed throughout their lives *(*Aceituno-Madera, Buendia-Eisman and Arias Santiago, 2010; de Gruijl, 1999).

According to the World Health Organization (WHO), from two to three million new cases of non-melanotic skin cancer and some 132 thousand cases of melanotic skin cancer are reported every year. Non-melanotic skin cancers can be surgically removed and are rarely fatal, but malignant melanomas substantially contribute to the mortality in light-skinned populations (WHO, 2003).

There are two main types of ultraviolet radiation that affect human health:
1. UV radiation with wavelengths between 290 and 320 nm. Approximately four percent of the total UV light on the earth surface. This is the portion with lower wavelengths, known as UVB. This radiation only manages to penetrate the outer skin layer causing reddening or "tanning" of the skin, and has been mainly associated to less severe diseases such as cancer types known as basal cell and squamous cell carcinomas *(von Thaler, Kamenish and Berneburg, 2010).*
2. UV radiation with wavelengths between 320 and 400 nm. This is around 96% of the total UV light that reaches the earth surface, known as UVA. This radiation penetrates the skin tissue at greater depth potentially causing damage to cellular DNA, and is the main cause of malignant melanoma *(Cadet, Douki and Ravanat, 2009*)*.*

The development of substances aiming at preventing skin darkening, sunspots, premature aging, and allowing higher exposure times or a lower risk of skin lesions has introduced a significant number of molecules that act as chemical absorbents or physical sunscreens intended to meet these needs.

In the state of the art various sun protection agents are described whose action mechanism is chemically induced. These are mainly aromatic organic compounds linked to other carbonyl groups that absorb UV radiation in a limited range of the spectrum and are the agents most frequently used. Others, more recently introduced into the market, are inorganic compounds that act as a physical obstacle reflecting or deflecting UV light rays and, in some cases, also partially absorbing a very specific range of radiation. Examples of substances in the chemical agent group include benzophenones, p-aminobenzoic acid (PABA) and its derivatives, cinnamates, avobenzone, among others. Up to this date, the family of inorganic agents is essentially limited to zinc oxide (ZnO) and titanium dioxide (TiO₂).

Taking into account the scientific and technological progress reached in the dermo-cosmetic field, it is difficult to explain that in spite of the number of people using sunscreens as a preventive measure, the incidence of cancer and other skin diseases continues increasing, and furthermore, there is evidence that among regular users of these products, the frequency of malignant melanoma (the type of skin cancer that causes more than 75% of deaths associated to this disease) continues on the rise (*Jemal, Thun and Ries, 2008; Osterlind, 1992*)*.*

It has been considered that the increase of these diseases in spite of the use of sunscreens may be due to the fact that some ultraviolet protection agents may be inducing the increasing damages observed. PABA and its derivatives, for example, once some of the active ingredients most widely used for ultraviolet protection, has shown in multiple surveys that may induce a damage mechanism on cellular DNA similar to that induced by UVA radiation (*Snyder and May, 1975; Flindt-Hansen, Thune and Eeg-Larsen, 1990*). There are other photoprotecting organic compounds which have been blamed of similar adverse effects. Therefore, over recent years the commercial attention was focused on inorganic agents such and ZnO and TiO₂.

Zinc oxide has been topically used as skin protector, scar tissue forming and anti-microbial agent for more than a century. Zinc oxide works by deflecting and reflecting UV and visible light. This mineral that provides UVB and UVA rays protection has anti-inflammatory properties and is considered a safe ingredient. However, the conventional form of zinc oxide leaves a white residue on the skin that is considered unpleasant by most people and aesthetically undesirable. For this reason, numberless research and development efforts have focused on obtaining ZnO nanoparticles that reach a higher degree of transparency. In like manner, titanium dioxide is a wide spectrum sun protection agent (providing UVA and UVB rays protection). However, the micronized or nanostructured form of this compound is crucial, since its whitening effect is much greater than that of ZnO. In both cases, there is concern on the toxicity of the nanoparticles in humans, still under investigation to this date (Sánchez, 2002). A study carried out by scientists of the Johnson Comprehensive Cancer Center of the University of California has shown that the nanoparticles of TiO₂ present in common toiletry products, such as cosmetics or suntan lotions, cause systemic genetic damage in mice. According to the results of this work, such nanoparticles induced breakages in ADN strains, causing chromosome damage and inflammation, and also increasing the risk of cancer in animals. Therefore, there is no certainty yet as if nanoparticles of inorganic agents used in sunscreens may --because of their size-- accumulate in various organs or go through the cell walls and interfere in intracellular mechanisms *(Schiestl, R, 2009).*

With respect to the types of adverse effects already identified in other sun protection agents, there is definitive evidence of mainly carcinogenic toxicity of many organic molecules and some of the inorganic molecules designed to absorb or block ultraviolet radiation. *(Dunford, 1997, Onuma, et al, 2009*)*.*

In the light of the state of the art, we know that the Food and Drug Administration (FDA), regulatory agency on public health matters in the United States of America, submitted a statement in 1999 reading to the letter: "The FDA has no knowledge of data showing that the mere use of ultraviolet protection prevents skin cancer." Furthermore, from June 2011, the FDA took measures to help protect consumers of skin damage caused by sun exposure regulating the claims of sunscreens manufacturers, that is, the degree of protection they offer along the different UV radiation ranges.

The Sun Protection Factor (SPF) or Protection Index is the first and most widely recognized measure to quantify photoprotection. It indicates the number of times that the photoprotector increases the capacity of natural defense of the skin against erythema or skin reddening prior to sunburn, so this is only providing information about the protection against UVB radiation. To estimate the SPF, the minimum dose of ultraviolet radiation that produces the first erythema reaction (or reddening of the skin) perceptible in human skin is assessed, that is, the minimum erythema dose (MED). MED is defined as the energy/time required for developing a minimum erythema in unprotected skin, after 24 hours of exposure. The SPF is the quotient by the energy required or the time required to produce an MED in protected skin, compared to unprotected skin (*Tendencias en Medicina, 2008*)*.*

Other major indicators of photoprotection include the UV Index related to the erythemal effects of sun radiation on the skin, the critical wavelength (pertaining to the wavelength for which the area under the integrated curve of optical density starting at 290nm is equal to 90% of the integrated area from 290 to 400 nm), and the UVA protection Index that allows us to assess the protection capacity of a sunscreen to prevent the effects of UVA rays. For this latter case, *in vivo* methods or *in vitro* methods are used. Some *in vivo* methods are the Immediate Pigmentation Index, the Persistent Pigmentation Index, the UVA Erythema Protection Factor, and the Phototoxic Protection Factor, among others. Among the *in vitro* methods are the Australian Standard AS/N75 2604.1997, the Boots Star Rating System, the APP/% Method of UVA protection, and the Diffey method, among others.

From what has been described supported in reports of the state of the art, sun protection agents developed and currently commercialized do not provide thorough safety and efficacy as required to prevent skin damages or cancer. The development of new compositions with safe and effective sunscreens is still required. Bismuth Oxychloride, an inorganic pigment accepted by international regulatory bodies such as the FDA in the United States, is here suggested as a new possible active. The FDA identifies this compound as a cosmetic pigmenting additive that does not require certification and is safe to be used in all types of personal care products, and even for ophthalmic use.

Bismuth Oxychloride is an inorganic chemical compound that has been used as pigment or filler in cosmetic compositions for many years. The US Patent 4,606,914 with priority date of October 26, 1981, describes the use of Bismuth Oxychloride as pigment in a cosmetic composition. In like manner, there are various patents describing several uses of Bismuth Oxychloride: as pearlescent pigment (US 3,980,491, US 4,820,510 and DE 2603211), and as pigment coating (US 4,076,551 and FR 2317909) among others.

Bismuth Oxychloride mainly works as a lustrous agent giving pearl-like effects imitating the aspect of natural pearls or shells. It is mainly used in prestigious cosmetics for very demanding markets since, unlike other titanated mica based pearlescent pigments, Bismuth Oxychloride allows a great diversity of sheen effects while providing the smoothness, silky feel and adherence required in skin applications. Furthermore, it helps to improve densification and integration of compact powder, loose powder and other cosmetic forms, and also to reduce the appearance of aging signs (wrinkling, age spots, etc.) without causing pore clogging.

In accordance with the chemical nature of Bismuth Oxychloride, notwithstanding it is a synthesized compound, it can also be classified as a "mineral", and this is why it has recently had major significance in so-called "mineral cosmetics". Bismuth --identified as the "green metal"-- presents superior properties concerning toxicity on its own, thus it can be used in numberless personal care, health care, and cosmetic applications *(Rohr, 2002; Palmieri, 1993*).

In the state of the art, Bismuth Oxychloride is found in patents that describe the use of this inorganic agent in: cosmetic compositions that protect the skin against IR and UV radiation, where Bismuth Oxychloride is exhibited as a substance reflecting IR radiation, while the UV light protection is conferred upon the liposoluble or hydrosoluble agents included in the composition (US Patent 5,000,937); a powder with Bismuth Oxychloride combined with a melanotic pigment used to pigment the skin and protect the skin against UV radiation, thus the melanotic pigment is in charge of the skin's UV protection (US Patent 5,205,837); cosmetic compositions that contain a dispersion of lipidic vesicles with a melanin pigment and other pigments (Bismuth Oxychloride among them) for UV protection of skin and/or hair (US Patent 5,874,091); cosmetic compositions with nanopigments with an optimum balance in UV protection/ transparency/ dispersibility, ideally, zinc oxide and titanium dioxide (US Patent 6,132,745 and US Patent 6,004,567); polymorphic lipidic particles as UV protection agents combined with organic and/or inorganic agents and pigments (US Patent 6,814,959).

This invention consists in the use of the inorganic compound known as Bismuth Oxychloride as a sun protection agent to be applied on the skin and/or hair, and/or facial or corporal hair, providing protection all along the UV radiation range.

### SUMMARY OF THE INVENTION

This novel invention refers to the use of a bismuth inorganic salt, more specifically, Bismuth Oxychloride, as a sun protection agent, and also to specific synthesis methods used to obtain Bismuth Oxychloride optimized with photoprotecting features ensuring ideal stability, effectiveness and safety to protect the skin and/or hair, and/or facial hair, and/or body hair from damage caused by UV sun radiation. Furthermore, different compositions are described with effective and safe concentrations of Bismuth Oxychloride, exhibiting higher protection values all along the ultraviolet spectrum (290 to 400 nm) than those commonly obtained with the organic and inorganic agents known in the state of the art. This compound allows critical wavelengths above 380nm which makes it a "wide spectrum" protection agent.

### BRIEF DESCRIPTION OF DRAWINGS

FIGURE 1. MPF-lambda curves for emulsions 5% active ingredient.
FIGURE 2. MPF-lambda curves for emulsions 10% active ingredient.
FIGURE 3. MPF-lambda curves for emulsions 15% active ingredient.
FIGURE 4. MPF-lambda curves for emulsions 20% active ingredient.
FIGURE 5. MPF-lambda curves for gels 20% active ingredient.
FIGURE 6. Comparative chart of the degree of protection of various compositions versus various compositions.

### DETAILED DESCRIPTION OF THIS INVENTION

The novel invention described below consists of: the use of a Bismuth inorganic salt, specifically Bismuth Oxychloride, as protection agent against solar ultraviolet radiation, as well as compositions with safe concentrations of Bismuth Oxychloride that in combination with excipients or vehicles known in the state of the art, work as skin and/or hair, and/or facial hair, and/or body hair protecting agents.

Surprisingly, Bismuth Oxychloride optimized according to this invention acts as an ultraviolet protection inorganic agent capable of reaching superior levels of sun protection compared to conventional organic and inorganic agents known in the state of the art.

Through the optimization of the Bismuth Oxychloride developed and described in this invention, a wide spectrum product capable of absorbing, reflecting or blocking ultraviolet sun radiation in the UV range (290 to 400 nm) was obtained. That is, this product provides UVA and UVB radiation protection, thus preventing damages caused thereby, such as: sun burn (erythema), skin tanning, acute actinic damage, chronic actinic damage, dermatoheliosis, dryness, pitted texture, loss of elasticity, stain alterations, wrinkles, epidermal acanthosis (enlargement), elastosis, proliferation of fiber blasts and mast cells, dilated and tortuous blood vessels, telangiectasias, degeneration of elastic fibers, photo-aging, immune-suppression, sun keratosis, non-invasive surface lesions, photosensitivity reactions, genetic deficiencies related dermatosis, skin pathologies that may be worsened by sun exposure and photodermatosis directly produced by sun exposure, lupus erythematosus, xeroderma pigmentosum, chronic actinic dermatitis, polymorphous light eruption, persistent light reaction, porphyrias, urticaria solaris, non-melanotic skin cancers (NMSC), basal cell carcinomas (BCC) and squamous cell carcinoma (SCC), the dangerous malignant, intracutaneous, invasive, metastatic and fatal melanotic cancer, among other diseases induced in the skin by sun radiation.

Another feature of this invention is that by using optimized Bismuth Oxychloride, sun radiation protection is higher than that obtained with products containing ultraviolet protection agents known in the state of the art, also showing higher safety and less adverse effects than the latter.

The ultraviolet protection agent described in this invention is made up of specific degrees of Bismuth Oxychloride synthesized in such a way that exhibit specific particle size distributions and stability levels that in a non-expected and surprising manner derive in high levels of sun protection and the capability of such agent of absorbing, reflecting and/or blocking the sun radiation all along the ultraviolet spectrum, preventing the absorption of such radiation by the skin and/or hair, and or facial hair, and or body hair.

The application of Bismuth Oxychloride as described in this patent may be used on its own or in combination with one or more vehicles, or one or more excipients known in the state of the art. The composition with Bismuth Oxychloride may be made in a descriptive but not limitative manner as water/oil emulsions, oil/water emulsions or their combinations, solutions, compact powders, lose powders, cold creams, gels, lotions, balms, pastes, waxes, eye drops, liniments, ointments, creams, aerosols and powders, among others, as well as in make-up form in liquids or emulsions, compact or lose powders and anhydrous bars or creams, polishes, hair dyes, shampoo, liquid soap and soap bars, as well as the combinations of one or more forms of preparation.

Bismuth Oxychloride shows good compatibility with other sun protection agents known in the state of the art thus may be combined with them or not. Among the sun protection agents known with which it may or may not be combined are, without limitation, the following: titanium dioxide, zinc oxide, benzophenone and its derivatives, 4 phenylbenzophenone, 2-hydroxy-4-n-octyloxi-benzophenone, 2-hydroxy-4-methoxy-benzophenone, 2,2'-dihydroxy-4, 4-dimethoxy-benzophenone, suliso-benzone, bencimidazol derivatives, phenyl-bencymidazol sulfonic acid, camphor derivatives, 3-benzyledene-camphor, 3-(4-methylbenzyledene-camphor, terephthaledene-dicamphor, sulfonic acid, dibenzoyl-methanes, 4-isopropyl-dibenzoyl-methane, 4-ter-buthyl -4'-metoxy-dibenzoyl-methane, cinnamic acid esters, 2-ethyl-hexyl p-methoxy cinnamic acid esters, p-methoxy-cinnamic isoamyl acid esters, p-methoxy-cinnamic octyl acid esters, p-methoxy-cinnamic propyl acid esters, p-aminobenzoic acid (PABA) and its derivatives, p-aminobenzoic glycerol acid esters, butyl-PABA, octyl-dimetyl-PABA, 2-ethyl-hexyl salicilate, homosalate, octyl salicilate, octyltriazone and oxybenzone, among others, and/or their combinations.

The action, safety, efficacy and effectiveness mechanisms exhibited by this novel composition of Bismuth Oxychloride allow the application thereof to be used to prevent skin and/or hair, and/or facial hair, and/or body hair damage of mammals, ideally humans, with minimum or null adverse effects, being even able to be used for the skin and/or hair protection of babies.

In an illustrative but not limitative manner, rheologic additives, dispersants, surfactants, emollients, polymers or any other material known in the state of the art and used for cosmetic and/or pharmaceutical and/or veterinary compositions may be incorporated or not to compositions with Bismuth Oxychloride.

The stability shown by the degrees of Bismuth Oxychloride useful as UV protection agents may be low, medium or high, that is, darkening is observed due to intemperism (particularly ultraviolet light), the medium to high degrees being ideal. Particularly, the Bismuth Oxychloride degrees developed that in an unexpected manner can obtain higher sun protection levels may exhibit stability by themselves or may be stabilized through common processes known in the state of the art. Furthermore, to improve its performance or to exhibit additional features such as higher degree of UV protection, better formulation characteristics, better appearance, tailored smoothness, among others, the Bismuth Oxychloride compound resulting from this invention may or may not become functionalized (surface modification, coating, co-precipitation, micro-encapsulation, among others existing in the state of the art).

The Bismuth Oxychloride obtained as photoprotecting agent typically shows optimum particle size distributions for the purpose of this invention, which may range from 0.01 microns up to 80 microns, but ideally from 0.2 to 20 microns, and even more so, from 0.3 to 5 microns. This control of particle size distribution is obtained through any of the chemical and physical methods for growth, reduction and/or separation of particles making up the product already known in the state of the art.

As part of obtaining the specific particle size of Bismuth Oxychloride resulting from this invention, controls of process variables were implemented, such as: the nature of the medium in which hydrolysis is made, pH, temperature, concentration of reagents, stirring speed, type and time, as well as the presence of other chemical species or catalysts.

### EXAMPLES

In an illustrative but not limitative manner, some examples of the various procedures to prepare Bismuth Oxychloride and compositions obtained during the development of this invention are presented herein below:

### Example 1. Degree of optimized Bismuth Oxychloride for UV protection with normal particle size distribution below 3 microns.

80 liters of a 1.9 molar solution of bismuth nitrate (Bi (NO₃)₃) are mixed to cause a reaction with 1000 liters of a 0.5 molar solution of HCl while keeping a pH control between 1 and 2 with the addition of a sodium hydroxide solution at 50%*. The Bismuth Oxychloride precipitate is separated by centrifugation and washed with ethylic alcohol at 50%. Excess moisture is withdrawn in a rotary drying process within a temperature range of 70°C-100°C. The product thus obtained shows a particle size normal distribution centered on 2.5 microns and a high light resistance.

### Example 2. Degree of optimized Bismuth Oxychloride for UV protection with normal particle size distribution below 1 micron.

17 liters of hydrochloric acid at 30% are added to 80 liters of a 1.7 M solution of bismuth nitrate (Bi (NO₃)₃). 1000 liters of a 0.3 M solution of hydrochloric acid are slowly added while stirring. The precipitate of Bismuth Oxychloride is separated by vacuum filtration while washed with ethylic alcohol at 50%. Excess moisture is withdrawn in a dryer (***de pantalón***) within a temperature range of 70°C-100°C. The product obtained has a particle size normal distribution centered on 0.7 microns, and a medium light resistance.

### Example 3. Water/oil emulsion.

A simple water/oil emulsion is prepared with the following composition:

| Component | Amount (% P/P) |
|---|---|
| PHASE A | |
| Glyceryl Monooleate | 5 - 10 |
| Mineral oil | 2 - 9 |
| Lanolinic alcohol | 2 - 5 |
| Glycerin | 4 - 8 |

| PHASE B | |
|---|---|
| Deionized water | 60 - 90 |
| Rheologic agent | 1 - 3 |

| PHASE C | |
|---|---|
| Sodium benzoate | 0 - 1 |

Phases A and B are separately heated within a temperature range of 70-80°C while stirring vigorously until an homogeneous gel is obtained in the first instance, and a uniform oily solution in the second. Phase B is slowly added to Phase A while stirring continuously until an emulsion is obtained. Cool at room temperature before adding Phase C.

Using the emulsion described, four inorganic UV protection agents were compared concerning their performance as sun protection active agents at different concentrations. The table below shows the code allocated to each of the cosmetic compositions prepared:

| Product | Concentration | | | |
|---|---|---|---|---|
| | 5% | 10% | 15% | 20% |
| Commercial pearlescent pigment of Bismuth Oxychloride (a)* | A5 | A10 | A15 | A20 |
| Commercial pearlescent pigment of Bismuth Oxychloride (b)* | B5 | B10 | B15 | B20 |
| Commercial nanometric inorganic active of ZnO** | C5 | C10 | C15 | B20 |
| Bismuth Oxychloride optimized as UV protection active | D5 | D10 | D15 | D20 |

| | | | | |
|---|---|---|---|---|
| *FarmaQuimia SA de CV **Elementis Specialties Inc. | | | | |

Sun protection properties were assessed using an Optometrics SPF-290S analyzer, following the FDA standard in-vitro procedure, with 2mL/cm2 on Transpore Tape^{®} in a quartz platen. Monochromatic protection factor (MPF) data were recorded along the UVB (290 to 320 nm) to UVA (320 to 400 nm) regions of the radiation spectrum at 2nm intervals. The SPF results were computed using standard sun radiation and erythemal constant values. The area under the curve, pertaining to total protection level, was also obtained by the instrumentation system package in all cases, used in this case to estimate the critical wavelength parameters, UVA/UVB, UVA I/UV relations, and Boots star ratings.

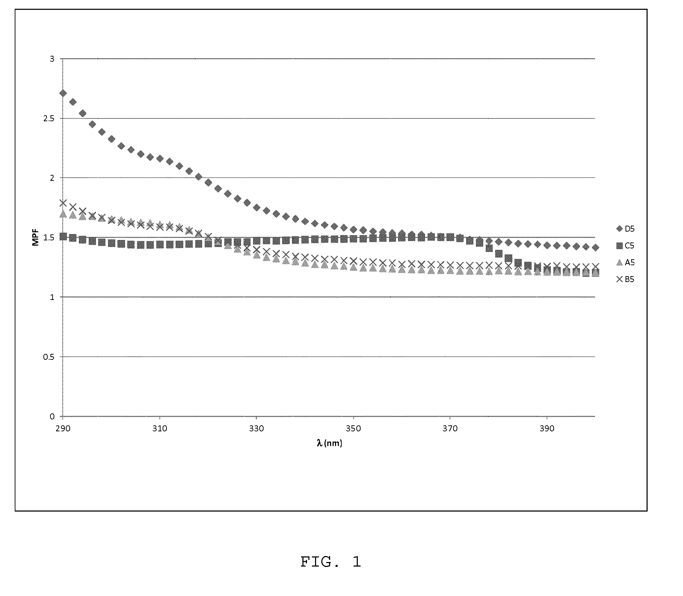

MPF-lambda curves for 5% active emulsions are presented in FIGURE 1 and a comparative summary of the sun protection parameters is presented in the table below:

| Properties | 5% Active Emulsion | | | |
|---|---|---|---|---|
| | A5 | B5 | C5 | D5 |
| SPF | 1.56 | 1.56 | 1.45 | 2.09 |
| UVA/UVB | 0.512 | 0.589 | 0.995 | 0.587 |
| UVA I/UV | 0.69 | 0.76 | 0.96 | 0.77 |
| UVA I/UV Grade | Medium | High | Very high | High |
| Critical Wavelength | 381.7 | 384.2 | 381.5 | 382.3 |
| Area under the curve | 14.32 | 15.47 | 16.81 | 25.83 |
| Boots Star Rating (2004)* | 2 | 2 | 5 | 2 |
| | Moderate | Moderate | Ultra | Moderate |

| | | | | |
|---|---|---|---|---|
| *The Boots Star Rating System is commonly used in Europe. However, the FDA has not approved it as a measurement system. Surveys continue to be performed to achieve its standardization. | | | | |

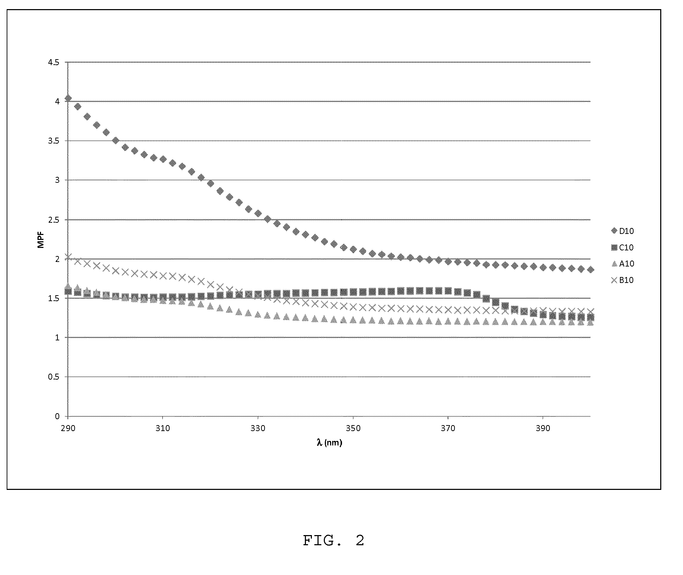

MPF-lambda curves for 10% active emulsions are presented in FIGURE 2, and a comparative summary of the sun protection parameters is presented in the table below:

| Properties | 10% Active Emulsion | | | |
|---|---|---|---|---|
| | A10 | B10 | C10 | D10 |
| SPF | 1.44 | 1. 74 | 1.52 | 3.10 |
| UVA/UVB | 0.544 | 0.6 | 1.0195 | 0.663 |
| UVA I/UV | 0.75 | 0.76 | 0.97 | 0.77 |
| UVA I/UV Grade | High | High | Very high | High |
| Max %T COV | 177 | 194 | 278 | 278 |
| Critical Wavelength | 383.1 | 384.1 | 382.05 | 384.3 |
| Area under the curve | 12.46 | 19.40 | 19.025 | 41.60 |
| Boots Star Rating (2004) | 2 | 3 | 5 | 3 |
| | Moderate | Good | Ultra | Good |

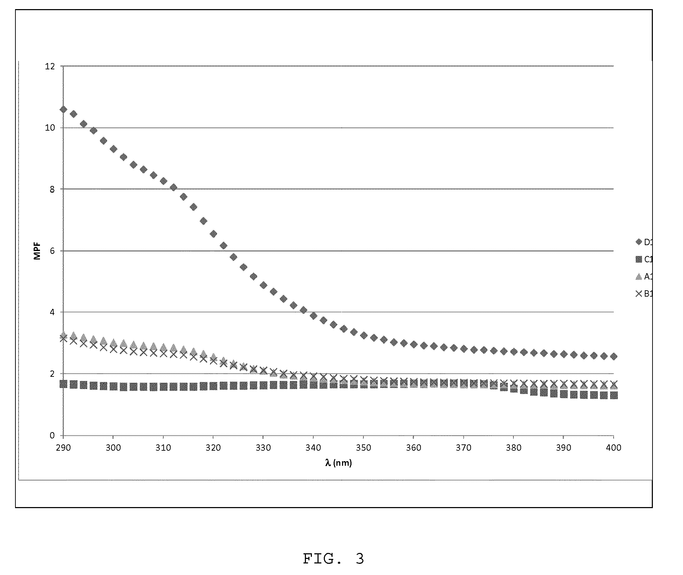

MPF-lambda curves for 15% active emulsions are presented in FIGURE 3, and a comparative summary of the sun protection parameters is presented in the table below:

| Properties | 15% Active Emulsion | | | |
|---|---|---|---|---|
| | A15 | B15 | C15 | D15 |
| SPF | 2.69 | 2.54 | 1.59 | 7.13 |
| UVA/UVB | 0.577 | 0.663 | 1.044 | 0.591 |
| UVA I/UV | 0.75 | 0.80 | 0.98 | 0.74 |
| UVA I/UV Grade | High | High | Very high | High |
| Critical Wavelength | 383.70 | 384.50 | 382.60 | 382.4 |
| Area under the curve | 33.78 | 33.42 | 21.24 | 69.01 |
| Boots Star Rating (2004) | 2 | 3 | 5 | 2 |
| | Moderate | Good | Ultra | Moderate |

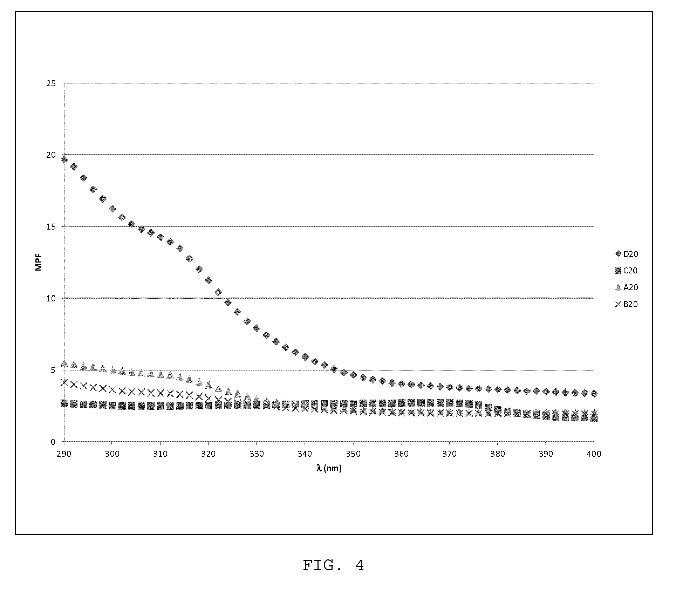

MPF-lambda curves for 20% active emulsions are presented in FIGURE 4, and a comparative summary of the sun protection parameters is presented in the table below:

| Properties | 20% Active Emulsion | | | |
|---|---|---|---|---|
| | A20 | B20 | C20 | D20 |
| SPF | 4.22 | 3.22 | 2.52 | 11.70 |
| UVA/UVB | 0.579 | 0.657 | 1.009 | 0.615 |
| UVA I/UV | 0.74 | 0.82 | 0.97 | 0.75 |
| UVA I/UV Grade | High | High | Very high | High |
| Critical Wavelength | 383.20 | 384.70 | 382.90 | 382.60 |
| Area under the curve | 49.49 | 42.61 | 41.02 | 87.46 |
| Boots Star Rating (2004) | 2 | 3 | 5 | 3 |
| | Moderate | Good | Ultra | Good |

### Example 4. Carbomer gel

A carbomer gel is prepared according to the following composition:

| Component | Quantity (% P/P) |
|---|---|
| Deionized water | 65 - 90 |
| Methacrylic acid derivative | 1 - 5 |
| Preservative | 0.0 - 0.1 |

Methacrylic acid derivative is added to deionized water and carefully mixed until a homogeneous gel is obtained. Preservative is added while stirring slowly.

Using a gel as the one described above, four UV protection inorganic agents were compared concerning their performance as sun protection actives at different concentrations. The table below shows the code allocated to each of the cosmetic compositions prepared:

| Product | Concentration 20% |
|---|---|
| TiO₂Commercial micronized inorganic active** | F20g |
| Optimized Bismuth Oxychloride as UV protection active (a) | D20g |
| Optimized Bismuth Oxychloride as UV protection active (b) | G20g |
| Optimized Bismuth Oxychloride as UV protection active (c) | H20g |

| | |
|---|---|
| **Kemira | |

Sun protection properties were assessed through an Optometrics SPF-290S analyzer, using the FDA standard in-vitro procedure, with 2mL/cm² on Transpore Tape^{®} in a quartz platen. Monochromatic Protection Factor (MPF) data were recorded all along the UVB (290 to 320 nm) and UVA (320 to 400 nm) regions of the spectrum at 2 nm intervals. SPF results were computed using sun radiation standard values and erythemal constants. The area under the curve pertaining to total protection level was also obtained through packages of the instrumentation system in all cases used to estimate critical wavelength parameters, the UVA/UVB, UVA I/UV relationship, and Boots rating.

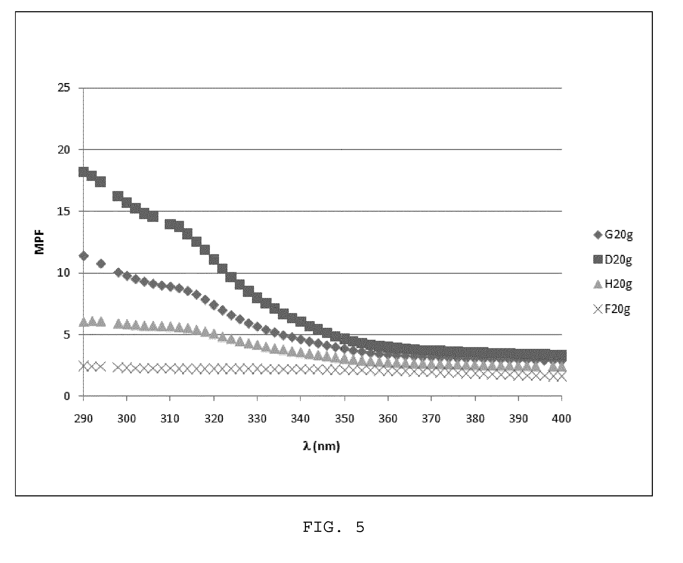

MPF-lambda curves for the 20% active carbomer gels are presented in FIGURE 5, and a comparative summary of the sun protection parameters is presented in the table below:

| Properties | 20% Active Gels | | | |
|---|---|---|---|---|
| | D20 | F20 | G20 | H20 |
| SPF | 11.54 | 2.26 | 7.80 | 5.20 |
| UVA/UVB | 0.617 | 0.897 | 0.644 | 0.679 |
| UVA I/UV | 0.75 | 0.90 | 0.77 | 0.78 |
| UVA I/UV Grade | High | High | High | High |
| Critical Wavelength | 382.30 | 384.6 | 383.8 | 384.3 |
| Area under the curve | 86.33 | 34.74 | 74.11 | 60.47 |
| Boots Star Rating (2004) | 3 | 5 | 3 | 3 |
| | Good | Ultra | Good | Good |

In the light of this invention and compared to the known inorganic agents (TiO₂, ZnO), the ultraviolet protection agents based on Bismuth Oxychloride and described in this invention exhibited a superior protection magnitude all along the UV radiation spectrum (290 nm to 400 nm), never reported before in the state of the art. To demonstrate the aforementioned and as we have already described, formulations prepared at same concentrations and in identical compositions with TiO₂ and ZnO commercial inorganic agents were compared to the Bismuth Oxychloride compositions described in this invention. The findings show a surprising superior protection magnitude all along the UV radiation spectrum (290nm to 400nm), not previously reported in the state of the art. This is ratified by the monochromatic protection factor curves (MPF) (Figures 1, 2, 3, 4 and 5) that show the amount of radiation blocked (either by absorption, blocking or reflection) at each particular wavelength. In one case, identical water-in-oil emulsions with 15% p/p of ultraviolet protection agent, exhibited a magnitude some five times higher in the UVB region when it is Bismuth Oxychloride versus a ZnO commercial ingredient. With respect to the UVA radiation protection magnitude, the protection obtained with Bismuth Oxychloride is twice higher than that of ZnO. In like manner, compositions based on carbomer gel with 20% of active ingredient resulted in protection magnitudes six times higher from the product with Bismuth Oxychloride compared to a TiO₂ commercial ingredient in the UVB region and twice higher for UVA. The aforementioned is based on the areas under the MPF curve for each case, and this value is directly proportional to the degree of protection. It is important to point out the protection difference observed in the UVA and UVB regions: although the degree of protection offered by optimized Bismuth Oxychloride are higher in both cases than conventional inorganic agents, UVB protection is significantly higher. Consequently, the UVA I/UV relationship and the Boot's Star Rating (based on the UVA/UVB fraction), are not consistent with the results obtained due to the fact that these measurement scales are based on TiO₂ and ZnO. In the light of this invention, such results strengthen the concern prevailing in the state of the art on the need of an adequate and standardized regulation to evaluate UVA protection.

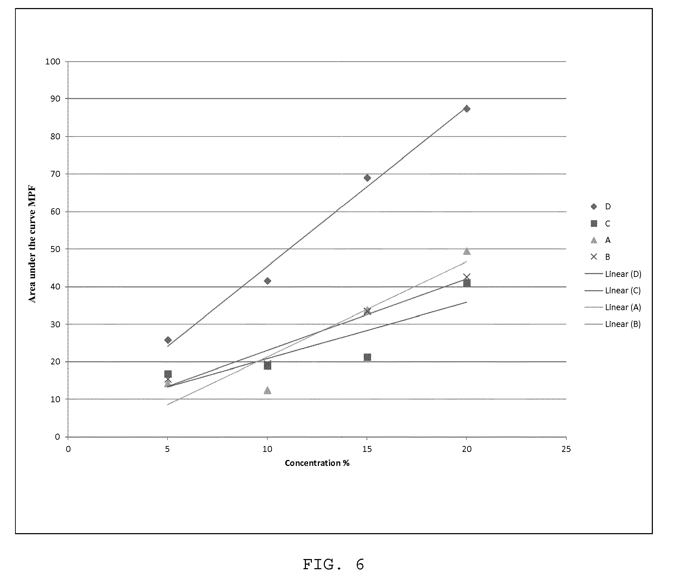

An additional feature of the degrees of Bismuth Oxychloride subject matter of this invention is the direct relationship of the degree of protection with the concentration thereof in a particular composition (FIGURE 6). As can be noted in the plot, the more the concentration is increased the higher the protection magnitude of Bismuth Oxychloride compared to other commercial protection inorganic agents. This allows formulation of dermo-cosmetic compositions with high protection degrees using a lower amount of active ingredient.

In a descriptive but not limitative manner, this invention offers the following advantages:
1. A novel sun protection inorganic agent for the skin and/or hair, and/or face hair, and or body hair.
2. The use of the inorganic salt of Bismuth Oxychloride for protecting the skin and/or hair, and or face hair, and/or body hair against sun radiation all along the Ultraviolet spectrum (290 to 400 nm).
3. A safe, effective and efficient sun protection inorganic agent approved for cosmetic and pharmaceutical use.
4. The pharmaceutically and cosmetically accepted inorganic salt of Bismuth Oxychloride susceptible of being used in compositions as a sun protecting agent.
5. The inorganic salt of Bismuth Oxychloride is pharmaceutically and cosmetically acceptable to prevent skin diseases.
6. Bismuth Oxychloride does not have any adverse effect compared to other known sun protecting agents.
7. Bismuth Oxychloride is aesthetically more acceptable compared to other inorganic sun protecting agents known.
8. The action mechanism of Bismuth Oxychloride is based on blocking and/or absorbing, and/or reflecting UV rays.
9. It can be used in mammals of any age.
10. The magnitude of UV protection of optimized Bismuth Oxychloride is significantly higher than that of other sun protection inorganic agents known in the state of the art.
11. The sun protection magnitude of Bismuth Oxychloride is directly proportional to concentration.
12. The protection magnitude of Bismuth Oxychloride increases with concentration compared to other commercial protection inorganic agents.
13. The Bismuth Oxychloride salt is compatible with human skin.

## Claims

1. The use of a Bismuth inorganic salt as a pharmaceutical and/or cosmetic sun protection agent for the care and/or prevention, and/or treatment of symptoms, and/or disorders, and/or diseases, and/or aesthetic conditions of the skin, and/or hair, and/or facial and/or body hair caused by sun radiation, alone or in combination of one or more vehicles and/or excipients, and/or additives.

2. The use pursuant to claim 1 of a Bismuth inorganic salt as pharmaceutical and/or cosmetic sun protection agent for the care and/or prevention, and/or treatment of symptoms, and/or disorders, and/or diseases, and/or aesthetic conditions of the skin and/or hair, and/or facial and/or body hair caused by sun radiation, alone or in combination with one or more vehicles and/or excipients, and/or additives, **characterized in that** this Bismuth inorganic salt is Bismuth Oxychloride and/or its pharmaceutically and/or cosmetically acceptable molecular forms.

3. The use of Bismuth Oxychloride pursuant to claims 1 and 2 **characterized in that** it prevents and/or avoids symptoms and/or disorders, and/or diseases, and/or aesthetic conditions of the skin and/or hair, and/or facial and/or body hair caused by ultraviolet sun radiation.

4. The use pursuant to claims 1 to 3 **characterized in that** the symptoms and/or disorders, and/or diseases, and/or aesthetic conditions of the skin are: sunburn (erythema), skin tanning, acute actinic damage, chronic actinic damage, dermatoheliosis, dryness, pitted texture, loss of elasticity, coloring alterations, wrinkles, epidermal acanthosis (swelling), elastosis, proliferation of fibroblast and mast cells, tortuous and dilated blood vessels, telangiectasias, elastic fiber degeneration, photoaging, immunosuppression, solar keratosis, non-invasive surface lesions, photosensitivity reactions, genetic deficiencies related dermatoses, skin diseases that may be worsened by the sun, photodermatosis directly caused by the sun, lupus erythematosus, xeroderma pigmentosum, chronic actinic dermatitis, light polymorphous eruption, light persistent reactions, porphyria disorders, sun rashes, non-melanotic skin cancers, basal cell carcinoma, spinal cell carcinoma, malignant, intradermal, invasive, metastatic and fatal melanotic cancer, among other skin diseases caused by sun radiation.

5. The use of Bismuth Oxychloride pursuant to claims 1 to 4 **characterized in that** Bismuth Oxychloride exhibits a particle size distribution from 0.01 microns to 100 microns.

6. The use of Bismuth Oxychloride pursuant to claims 1 to 5 **characterized in that** Bismuth Oxychloride may be added to water/oil or oil/water emulsions, or their combinations, solutions, compact powders, lose powders, cold creams, gels, lotions, balms, pastes, waxes, collyria, ophthalmic drops, lotions, liniments, ointments, creams, aerosols, bars, capsules, tablets, foams, vaporizers, patches, suspensions, salts, pearls, vials, enamels, dyes, tinctures, shampoos, liquid and/or solid soaps, liposomes, as well as to combinations of one or more forms.

7. Pharmaceutical and/or cosmetic compositions pursuant to claims 1 to 6, **characterized in that** they exhibit a Sun Protection Factor within a range of 1 to 50.

8. Pharmaceutical and/or cosmetic compositions pursuant to claims 1 to 7 **characterized in that** they exhibit a UVA/UVB Factor of at least 0.4.

9. Pharmaceutical and/or cosmetic compositions pursuant to claims 1 to 8, **characterized in that** they exhibit a UVA I/UV Factor of at least 0.6.

10. Pharmaceutical and/or cosmetic compositions pursuant to claims 1 to 9 **characterized in that** they exhibit a UV I/UV Factor from MEDIUM to VERY HIGH Grade.

11. Pharmaceutical and/or cosmetic compositions pursuant to claims 1 to 10 **characterized in that** they exhibit a Critical Wavelength higher than 370nm.

12. The use of Bismuth Oxychloride pursuant to claims 1 to 11 as pharmaceutical and/or cosmetic sun protection agent for the care and/or prevention, and/or treatment of symptoms and/or disorders, and/or diseases, and/or aesthetical conditions of the skin and/or hair, and/or facial and/or body hair caused by sun radiation, alone or in combination with one or more vehicles and/or excipients, and/or additives **characterized in that** Bismuth Oxychloride is functionalized and/or modified, and/or in nanoparticulated systems.

13. The use of Bismuth Oxychloride pursuant to claims 1 to 12 alone or in combination with one or more inorganic and/or organic sun protection agents.
